# EUROPEAN PATENT SPECIFICATION

(11) **EP 3 554 384 B1**
(45) Date of publication and mention of the grant of the patent: **14.09.2022**
(21) Application number: 17835826.3
(22) Date of filing: 18.12.2017
(51) Int. Cl.: A61B 8/08, A61B 8/00

(54) **FETAL ULTRASOUND IMAGING**
FÖTALE ULTRASCHALLBILDGEBUNG
IMAGERIE F TALE PAR ULTRASONS

(30) Priority: 19.12.2016 EP 16306720
(43) Date of publication of application: 23.10.2019
(73) Proprietor: Koninklijke Philips N.V., 5656 AG Eindhoven (NL)
(72) Inventor: CIOFOLO-VEIT, Cybèle, 5656 AE Eindhoven (NL); PERROT, Matthieu, 5656 AE Eindhoven (NL); ROUET, Jean-Michel, 5656 AE Eindhoven (NL)
(74) Representative: Philips Intellectual Property & Standards
(86) International application number: PCT/EP2017/083269
(87) International publication number: WO 2018/114774

(56) References cited:
- WO-A1-2010/046819
- CN-A- 105 433 988
- JP-A- 2016 036 594
- US-A1- 2007 255 139
- US-A1- 2010 099 987
- US-A1- 2012 232 394
- US-A1- 2015 262 353
- US-A1- 2016 058 422

## Description

### FIELD OF THE INVENTION

The present invention relates to an ultrasound imaging system comprising a data storage device for storing ultrasound imaging data comprising an imaging volume of a fetus; a processor arrangement communicatively coupled to the data storage device and responsive to a user interface; and a display device under control of said processor arrangement.

The present invention further relates to a method of visualizing a volume slice of an imaging volume of a fetus with such an ultrasound imaging system.

The present invention further relates to a computer program product comprising a computer readable storage medium having computer readable program instructions embodied therewith for, when executed on the processor arrangement of such an ultrasound imaging system, cause the processor to implement such a method.

### BACKGROUND OF THE INVENTION

Ultrasonic imaging is routinely used during pregnancy to assess the development of a fetus in the mother's womb, for example to detect structural anomalies in the fetus. The traditional way for a clinician to acquire an image of each required view of the fetus is to manipulate an ultrasound probe while in acoustic contact with the abdomen of the mother until a desired anatomical orientation is in the plane of the 2D imaging probe. If multiple views are to be generated with such a procedure, there is a risk of missed abnormalities because obtaining and analyzing these views requires high skill (e.g. fetal echocardiography is very operator-dependent) whilst in addition the fetus may be moving during the procedure, requiring the clinician to reorient himself or herself with the fetus each time the fetus moves.

With the advent of three dimensional (3D) ultrasound image acquisition, it is now possible to capture a large volume of the fetus and to perform computed reconstruction of 2D views at any point in time, e.g. even after the patient (the fetus) is released. The 3D acquisition procedure may be conducted by making a slow sweep of the 2D image plane over the fetus or by electronically steering the ultrasound beams over the fetus. User-directed image processing may then be used for evaluation of the captured image volume, i.e. to evaluate the fetal anatomy. As such, 3D ultrasound image acquisition is less operator-dependent and facilitates evaluation of the image volume along different views, for example to answer different diagnostic questions following the examination of the fetus.

Of particular interest to analyse the development of the fetus are so-called biometry measurements, which are used to check if the fetus is developing correctly, e.g. within expected tolerances. Such biometry measurements may rely on an articulated fetus model, which is fitted to the 3D volume to facilitate the biometry measurements. However, the provision of such an articulated fetus model is a time-consuming exercise, as it requires prior detection and labelling of anatomical features such as bones and joints in the body of the fetus in order to provide target landmarks for the adjustment of the model position when fitted to the 3D volume. This is because automated labelling typically is difficult due to the fetal image being impaired by well-known artefacts, scattering and shadowing effects that result from its location in the mother's womb.

WO 2013/105815 A1 discloses an image processing apparatus including an image receiver which receives a predetermined image obtained by photographing a fetus; and a controller which detects a head region and a torso region of the fetus from the predetermined image, and which models a shape of the fetus by using at least one of a first contoured shape corresponding to the detected head region, a second contoured shape corresponding to the detected torso region, a first axis that is the central axis of the detected head region, and a second axis that is the central axis of the detected torso region, to model the fetus so that biometric data of the fetus can be easily measured. However, this apparatus does not assist its user in facilitating a labelling task of (other) anatomical features of the fetus in the 3D image.

WO 2010/046819 A1 discloses an ultrasound imaging system including an ultrasound scanning assembly providing volume data resulting from a three-dimensional scan of a body. A feature extractor searches for a best match between the volume data and a geometrical model of an anatomical entity. The geometrical model comprises respective segments representing respective anatomic features. Accordingly, the feature extractor provides an anatomy-related description of the volume data, which identifies respective geometrical locations of respective anatomic features in the volume data.

US 2012/0232394 A1 discloses an ultrasound diagnostic apparatus including a three-dimensional data generation unit which generates three-dimensional data for each region in the body of a subject based on reflected waves reflecting back from the body of the subject after ultrasound waves have been transmitted towards the body of the subject; a measurement image selection unit which respectively selects, for each region, one of the two-dimensional cross-sections that compose the three-dimensional data, as a measurement reference image used for measuring a length of each region in the body of the subject; a measurement calculation unit which measures a length of each region in the body of the subject using the respectively selected measurement reference image, and calculates an estimated weight of the subject using the measured lengths; and a display unit which outputs the estimated weight thus calculated.

### SUMMARY OF THE INVENTION

It The present invention is defined in the appended independent claims. seeks to provide ultrasound imaging system comprising a data storage device for storing ultrasound imaging data comprising an imaging volume of a fetus; a processor arrangement communicatively coupled to the data storage device and responsive to a user interface; and a display device under control of said processor arrangement that can be used to facilitate the labelling of anatomical features of a fetus.

The present invention further seeks to provide a method of visualizing a volume slice of an imaging volume of a fetus with such an ultrasound imaging system to facilitate the labelling of anatomical features of a fetus.

The present invention still further seeks to provide a computer program product comprising a computer readable storage medium having computer readable program instructions embodied therewith for, when executed on a processor arrangement of such an ultrasound imaging system, cause the processor arrangement to implement this method.

According to an aspect, there is provided an ultrasound imaging system comprising a data storage arrangement for storing ultrasound imaging data comprising an imaging volume of a fetus; a processor arrangement communicatively coupled to the data storage arrangement and responsive to a user interface; and a display device under control of said processor arrangement, wherein the processor arrangement is adapted to identify a a fetal reference structure having an elongated shape in said imaging volume; define a reference point on said fetal reference structure; interpret a translation instruction from the user interface as a translation of said reference point along the elongation direction of the identified fetal reference structure; display the imaging volume together with a volume slice of the imaging volume perpendicular to the elongation direction on the display device, said volume slice coinciding with the translated reference point, and visualize said translation along said elongated shape of the fetal reference structure in the displayed imaging volume.

The present invention is based on the insight that certain anatomical features of a fetus can be automatically recognised, e.g. using feature recognition algorithms executed by the processor arrangement, such as for example the spine of the fetus. Moreover, it has been realized that certain other fetal anatomical features may have a more or less fixed relationship to the fetal reference structure. Consequently, by restricting the user of the ultrasound imaging system to select volume slices of the imaging volume along the fetal reference structure, visualisation of such other fetal anatomical features is facilitated. The processor arrangement is adapted to display said volume slice together with said imaging volume on the display device to further aid the intuitive interpretation of the volume slices selected by the user.

In a preferred embodiment, the processor arrangement is further adapted to recognize a potential anatomical feature of said fetus in the volume slice coinciding with the translated reference point; and highlight the recognized potential anatomical feature in the displayed volume slice. For example, in case of a reference structure such as the spine, further anatomical features that are connected to the spine can be detected in a volume slice in a straightforward manner, as such further anatomical features typically have a comparable echogenicity to the spine such that their intensity in the volume slice will be comparable to that of the spine. In addition, such further anatomical features typically have a specific anatomical arrangement with respect to the main orientation/direction of the elongated reference structure. For example, shoulders, ribs and hips are more or less symmetrical with respect to a spine, and are connected to the spine under a specific angular orientation. Such prior knowledge may help to detect these further anatomical features, e.g. in order to highlight them in the displayed volume slice. In such a scenario, the processor arrangements may be adapted to identify regions in the volume slice that are adjacent to the reference structure and that have an intensity variation within a defined threshold compared to the intensity of the reference structure and/or may be adapted to identify regions having an expected geometrical relationship to the elongated reference structure, e.g. an expected symmetry and/or angular orientation as explained above. Such highlighted recognized potential anatomical features immediately draw the attention of the user of the ultrasound imaging system, thereby facilitating the labelling of the recognized potential anatomical features by the user if recognized by the user as an actual anatomical feature.

For example, the recognized potential anatomical feature may be highlighted by a colour overlay over said recognized potential anatomical feature to draw the attention of the user to the recognized potential anatomical feature. Such a colour overlay may be chosen in accordance with a colour convention in which different types, e.g. different shapes, of recognized potential anatomical features may be given a different colour overlay such that the user is furthermore presented with a suggestion of what the recognized potential anatomical feature may be, thereby further assisting the user in identifying anatomical features in the respective volume slices selected by the user.

Preferably, the processor arrangement is further adapted to receive a user identification of the recognized potential anatomical feature; and updating the volume slice with the user identification. For example, the user may use a keyboard or the like of the user interface to provide an identification of a recognized potential anatomical feature for example by pressing a single key on the keyboard, e.g. 'R' for ribs, 'H' for hips, 'S' for shoulder blades and so on or by selecting the appropriate identification from a menu such as a drop-down menu presented to the user on the display device. In this manner, the user may rapidly identify actual anatomical structures in the volume slices for labelling, e.g. to define anatomical landmarks within the imaging volume.

Such identified anatomical landmarks may be used by the processor arrangement to fit an articulated fetus model to said imaging volume on which the biometric evaluation of the fetus may be based.

The ultrasound imaging system may further comprise an ultrasound probe for obtaining the imaging volume of the fetus. Such an ultrasound probe may comprise transducer elements arranged in a two-dimensional (2D) array constructed to electronically steer ultrasound beams within the volumetric imaging region such that a three-dimensional ultrasound image frame of said region is provided. Alternatively, the array may be a one-dimensional array (1D) constructed to be mechanically steered through the volumetric imaging region in order to provide a three-dimensional ultrasound image frame.

According to another aspect, there is provided a method of visualizing a volume slice of an imaging volume of a fetus with an ultrasound imaging system, the method comprising retrieving said imaging volume from a data storage device; identifying a fetal reference structure having an elongated shape in said imaging volume; defining a reference point on said fetal reference structure; interpreting a translation instruction from the user interface as a translation of said reference point along the elongation direction of the identified elongated fetal reference structure; displaying the imaging volume together with a volume slice of the imaging volume perpendicular to the elongation direction, said volume slice coinciding with the translated reference point, and visualizing said translation along said elongated shape of the fetal reference structure in the displayed imaging volume. As explained above, this facilitates visualisation of such fetal anatomical features.

The method may further comprise displaying said volume slice together with said imaging volume to further aid the interpretation of the displayed ultrasound images by the user.

The method may further comprise recognizing a potential anatomical feature of said fetus in the volume slice coinciding with the translated reference point; and highlighting the recognized potential anatomical feature in the displayed volume slice to assist a user in identifying actual anatomical features in the displayed volume slice.

The method may further comprise highlighting the recognized potential anatomical feature by a colour overlay over said recognized potential anatomical feature to draw the attention of the user to the recognized potential anatomical feature.

In a preferred embodiment, the method further comprises receiving a user identification of the recognized potential anatomical feature; and labelling the volume slice with the user identification such that the labels may be used as anatomical landmarks for fitting an articulated fetus model to the imaging volume.

According to yet another aspect, there is provided a computer program product comprising a computer readable storage medium having computer readable program instructions embodied therewith for, when executed on a processor arrangement of an ultrasound imaging system according to any of the embodiments described in this application, cause the processor arrangement to implement the method according to any of the embodiments described in this application. Such a computer program product may be used to upgrade existing ultrasound imaging systems for fetal imaging such that these ultrasound imaging systems may implement one or more embodiments of the present invention.

### BRIEF DESCRIPTION OF THE DRAWINGS

Embodiments of the invention are described in more detail and by way of non-limiting examples with reference to the accompanying drawings, wherein:
Fig. 1 schematically depicts an ultrasound imaging system according to an example embodiment in operation;
Fig. 2 schematically depicts an aspect of the ultrasound imaging system of Fig. 1 in more detail;
Fig. 3 schematically depicts the principles of an articulated fetus model that can be used in biometric analysis of a fetus;
Fig. 4 schematically depicts a user interaction aspect of an embodiment of the present invention as displayed on a display device;
Fig. 5 is a flowchart of an example embodiment of the method of the present invention; and
Fig. 6 schematically depicts a block diagram of an example embodiment of an ultrasound imaging system of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

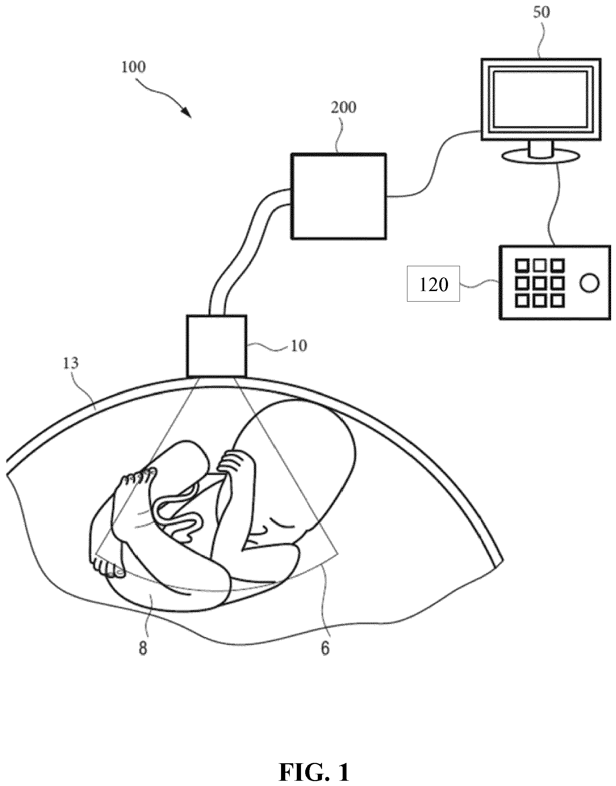

Fig. 1 shows a schematic illustration of an ultrasound imaging system 100 according to an example embodiment. The ultrasound imaging system 100 is applied to inspect a volumetric region of an anatomical site, in particular an anatomical site of a patient 13 including a fetus 8. The 3-D image of such a volumetric region will also be referred to as the imaging volume, whilst a 2-D slice of such a 3-D image will also be referred to as a volume slice.

The ultrasound imaging system 100 comprises an ultrasound probe 10 having at least one transducer array having a multitude of transducer elements for transmitting and/or receiving ultrasound waves. The transducer elements are preferably arranged in a two-dimensional (2D) array, which is constructed to electronically steer ultrasound beams within the volumetric region such that a three-dimensional ultrasound image frame of said region is provided. Alternatively, the array may be a one-dimensional array (1D) constructed to be mechanically steered through the volumetric region in order to provide a three-dimensional ultrasound image frame. The probe 10 is adapted to transmit ultrasound waves in a particular direction and to receive ultrasound waves from a particular direction which forms a field of view 6 for a given 3D image frame of the ultrasound probe 10. Such 3-D imaging is well-known per se and will therefore not be explained in further detail for the sake of brevity only.

In the embodiment shown in Fig. 1, the patient 13 is a pregnant person, wherein an anatomical entity to be inspected is a fetus 8, at least part of which is disposed in the field of view 6.

The ultrasound imaging system 100 further comprises an ultrasound imaging apparatus 200 such as a control unit, which typically comprises a processor arrangement 210 including one or more processing elements, and controls the provision of an ultrasound image via the ultrasound system 100. As will be explained further below, the ultrasound imaging apparatus 200 may receive ultrasound image data from the transducer array of the ultrasound probe 10 and provides a compounded three-dimensional (3D) ultrasound image derived from the different ultrasound data sets of the fetus 8.

The ultrasound imaging system 100 further comprise a display device 50 for displaying the ultrasound image received from the ultrasound imaging apparatus 200. Still further, a user interface 120 is provided that may comprise any combination of keys or a keyboard and inputting devices and may be connected to the display device 50 and/or directly to the ultrasound imaging apparatus 200. Such inputting devices for example may include a mouse, trackball, or the like. Other suitable inputting devices will be immediately apparent to the skilled person. In the context of the present application, a user may convey a translation instruction to the ultrasound imaging apparatus 200 by moving an input device such as a trackball or mouse, by clicking a key, and so on. It should be understood that a translation instruction in some embodiments equates to the movement of an input device such as a trackball or mouse by the user.

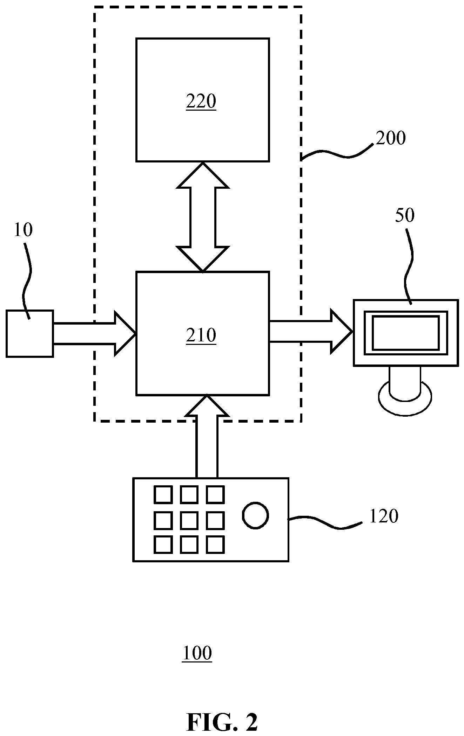

An example embodiment of the ultrasound imaging apparatus 200 is provided in more detail in Fig. 2, in which the ultrasound imaging apparatus 200 comprises at least a processing arrangement 210 and a data storage arrangement 220. The display device 50 may be separate to the ultrasound imaging apparatus 200 or may form part of the ultrasound imaging apparatus 200. Similarly, at least part of the user interface 120 may be separate to the ultrasound imaging apparatus 200 or may form part of the ultrasound imaging apparatus 200.

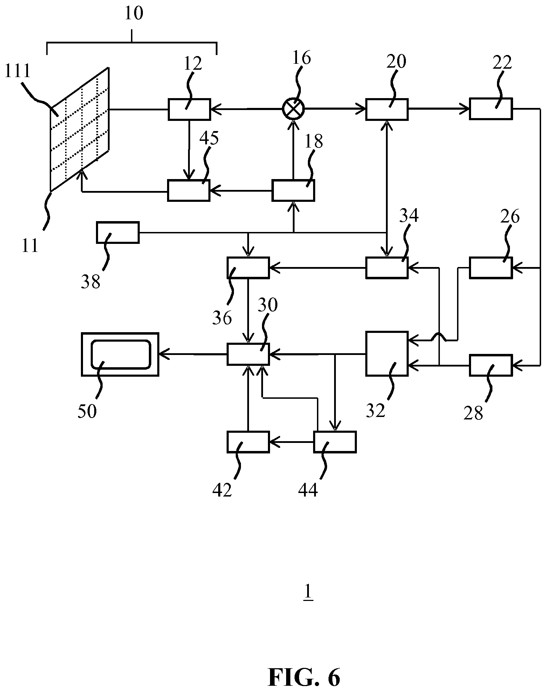

The processing arrangement 210 may include an ultrasound image processor adapted to process digital echo signals by spatial compounding in an ultrasound image processor, such as the ultrasound image processor 30 in the example embodiment of the ultrasound imaging system as schematically depicted in Fig. 6, which will be described in more detail below. The data storage arrangement 220 may comprise one or more memory devices, which may be discrete memory devices or may form part of the processing arrangement 210. For example, the data storage arrangement 220 may include a compound image memory unit, which may form part of the ultrasound image processor or may be separate to the ultrasound image processor. The compound image memory may be implemented as a 3D frame storage buffer and may be implemented as a dual port memory which can be written to and read from simultaneously. The use of such a R/W memory enables newly acquired 3D ultrasound image frames by the transducer array of the ultrasound probe 10 and the beamformer (to be described in more detail below) to be written into one area of the R/W memory while the data of other 3D image frames previously stored in the memory is read out and analyzed. The writing of new slice image data into the memory may be controlled by a write address controller while the reading of slice image data from other locations in the memory may be under the control of a read address controller, thereby facilitating real time image analyses and compounding. Of course, such a compound image memory unit equally may be used for evaluation of the imaging volume upon completion of its acquisition, e.g. after investigation of the patient.

The data storage arrangement 220, e.g. the compound image memory unit, may further store an articulated fetus model. The articulated fetus model takes into account the variability in overall orientation and articulated constellation of the fetal body. Such a model covers the most important articulation of the fetal skeletal anatomy and defines the degrees of freedom per articulation including range of motion (min/max angles). The implementation of the model is commonly done using joint (j)-limb (L)-relationships. By defining the common fetal structure a three-dimensional data set comprising a fetus can be segmented.

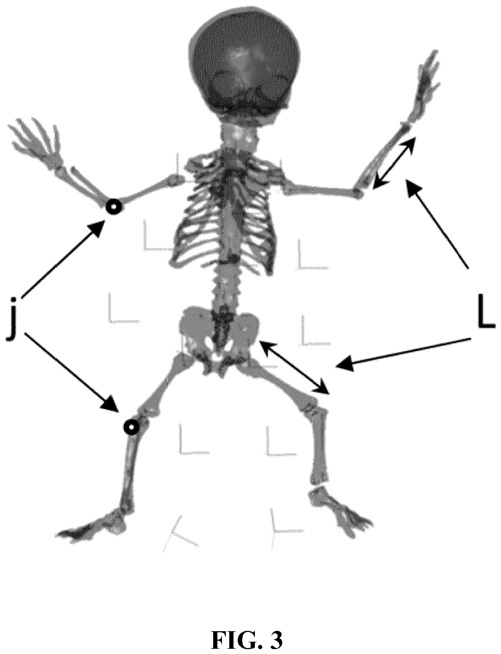

The idea is illustrated in Fig. 3 on a synthetic example. The articulated fetus model gives for each joint the respective articulation parameters (rotation point and angles) and thus defines the overall constellation. In this model following assumptions may be used. Each limb carries a shape, each limb can be saleable, can have a parent joint, and can have a list of child-joints. Each joint can be a hinge or a ball joint, has a static limb (with respect to this joint), and has a flexible limb. Thus, articulation can be defined by a rotation point, rotation axes and min/max of angulation values. The fetus position is given by the coordinate systems which define the location and rotation parameters of the respective joints. Based on this input, the actual fetus pose position (articulation) in the medical image can be determined using inverse kinematics to fit the desired position. When solving the inverse problem the degrees of freedom are considered per joint.

The ultrasound image processor of the processor arrangement 210 may comprise a segmentation unit arranged to segment each 3D image frame based on the articulated fetus model stored in the data storage arrangement 220. The segmentation unit thereby provides a plurality of 3D images originating from the plurality of 3D frames of the volumetric region, wherein a relative orientation of the fetus with respect to the look direction is identified for each 3D image. The relative orientation of the fetus linked to the 3D image gives a spatial relationship between the acquired 3D frames. As previously explained, the mapping of articulated fetus model onto the imaging volume including the fetus or part thereof typically requires alignment of reference points within the model with identified anatomical landmarks in the imaging volume. The provision of such identified anatomical landmarks in an automated fashion is far from trivial due to the fetal image being impaired by well-known artefacts, scattering and shadowing effects that result from its location in the mother's womb as previously explained. Consequently, such anatomical landmarks typically need to be provided manually by a user, but this is a cumbersome and time-consuming exercise.

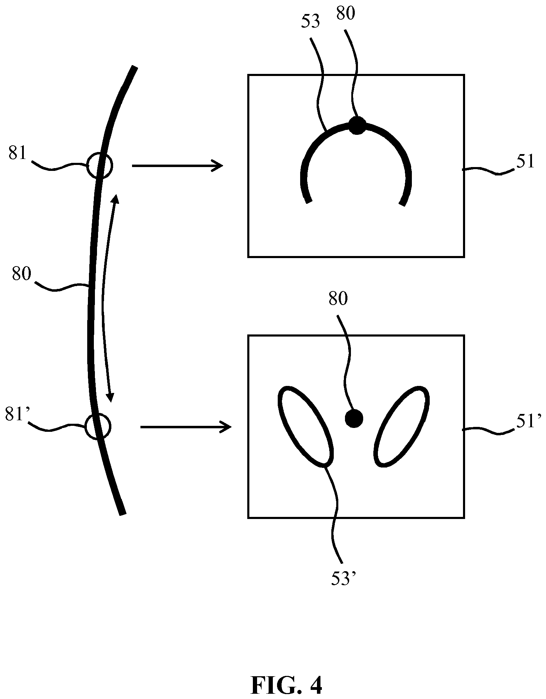

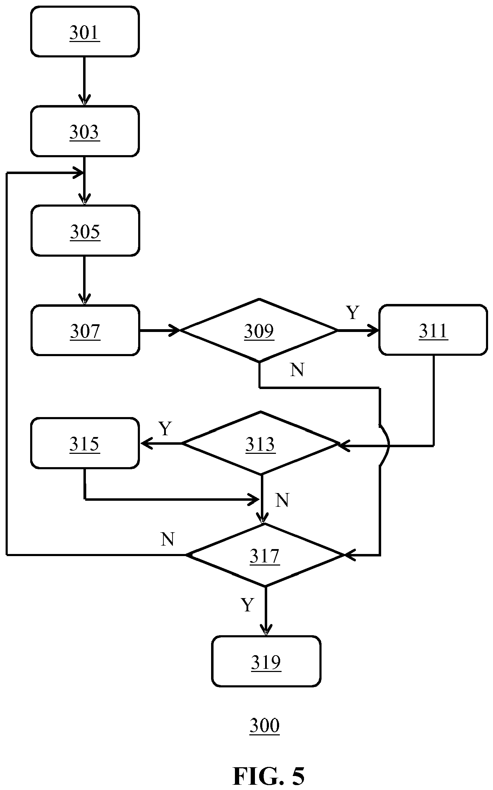

At least some embodiments of the present invention are aimed at facilitating the manual generation of such anatomical landmarks within the imaging volume. This is schematically depicted in Fig. 4, in which 2-D slices (volume slice) as displayed on the display device 50 are depicted in combination with the selection method of these slices in accordance with embodiments of the present invention, which method will be explained in further detail with the aid of Fig. 5 in which a flowchart of an example embodiment of a method 300 of visualizing a volume slice 51, 51 of an imaging volume of a fetus with an ultrasound imaging system, which method 300 may start in 301, e.g. by capturing the imaging volume and storing it in the data storage arrangement 220. For example, a plurality of 3D ultrasound image frames of the volumetric region (imaging volume) comprising a fetus may be acquired. Each 3D ultrasound image frame may be acquired at a different look direction with respect to the fetus.

In accordance with embodiments of the present invention, the processor arrangement 210 is adapted to identify a fetal reference structure 80 having an elongated shape in the imaging volume captured with the ultrasound probe 10. This for example may correspond to 303 in the method 300. To this end, the processor arrangement 210 may include an automated detection module (not shown) for detecting the fetal reference structure 80, which detection module may be a dedicated hardware component of the ultrasound imaging apparatus 200, which dedicated hardware component may form part of the processor arrangement 210, or alternatively this detection module may be realized in software for execution on a suitably configured processor of the processor arrangement 210, e.g. in the form of computer program instructions such as algorithms that cause such a suitably configured processor to identify the fetal reference structure 80 in the imaging volume. In an embodiment, the fetal reference structure 80 is the spine of the fetus. Such a reference structure may be detected using any suitable detection algorithm, e.g. a segmentation algorithm as is well-known per se to the skilled person. Automatic detection of the fetal spine has the further advantage that many anatomical features, e.g. skeletal structures, are positioned in the vicinity of the spine and/or are connected to the spine such that such anatomical features may be more readily identified when using the spine as a reference as will be explained in further detail below. However, it should be understood that the principles of embodiments of the present invention as explained in further detail below may be equally applied to other elongated fetal reference structures that can be automatically detected in an imaging volume captured with an ultrasound probe 10.

In accordance with the present invention, the identified fetal reference structure 80, which typically is an elongated structure such as the spine as explained above, is used by the ultrasound imaging apparatus 200 in 303, i.e. by the processor arrangement 210, to restrict the degrees of freedom by which a user can evaluate the imaging volume captured with the ultrasound probe 10. Specifically, the processor arrangement 210 is configured to interpret a translation instruction received from the user interface 120 in 305 as a translation of a reference point 81 along the identified fetal reference structure 80. The reference point 81 may be defined by the processor arrangement 210 upon identification of the fetal reference structure 80 in any suitable location along the fetal reference structure 80. In other words, any suitable starting point for the visualization of volume slices 51, 51' along the fetal reference structure 80 may be chosen by the processor arrangement 210 from which a translation instruction as received from the user interface 120 along the fetal reference structure 80 may be initiated, e.g. to arrive at a further reference point 81'. In this manner, a user may select a 2D volume slice 51, 51' along the fetal reference structure 80 with the user interface 120 in 305, e.g. by a movement or scrolling action with a mouse, trackball, or the like, which movement, scrolling action or similar causes the migration of the reference point 81 in any suitable direction along the fetal reference structure 80.

For each reference point 81, 81', e.g. each point along the fetal reference structure 80 selected by the user, the processor arrangement 210 extracts in 307 a volume slice 51, 51' from the imaging volume at the reference point 81, 81', which volume slice is in a plane oriented perpendicularly to the plane of the fetal reference structure 80 at the reference point 81, 81' and controls the display device 50 to display this volume slice. In an embodiment, the volume slice 51, 51' may be displayed together the imaging volume including the fetal reference structure 80 such that the user can define the desired reference point in reference to the fetal reference structure 80 as displayed in the imaging volume, for example by scrolling along the fetal reference structure 80 as displayed in the imaging volume and bring up perpendicular volume slices 51, 51' for landmark identification in a single view. To this end, the processor arrangement 210 translates a translation instruction as received from the user interface 120 as a translation visible in the imaging volume, e.g. as a cursor or the like tracking along the fetal reference structure 80 in accordance with the received translation instruction from the user operating the user interface 120.

In a preferred embodiment, the processor arrangement 210 is further adapted to evaluate each volume slice 51, 51' corresponding to a reference point 81, 81' in 309 in order to recognize potential anatomical features 53, 53' in these volume slices. For example, the processor arrangement 210 may be adapted to recognize image regions having a certain contrast ratio with surrounding image regions comparable to the contrast ratio between the cross-section of the fetal reference structure 80 in the volume slice 51, 51' and surrounding areas of the image. Alternatively, the processor arrangement 210 may be adapted to recognize image regions as potential anatomical features 53, 53' by identifying image regions adjacent to the cross-section of the fetal reference structure 80 that have the same intensity or brightness as the fetal reference structure 80. This for example is a particularly suitable approach where the fetal reference structure 80 is the spine given that the anatomical features adjacent to the spine typically are bone structures having the same or a similar echogenicity as the spine such that such anatomical features will have a comparable intensity or brightness to the spine. Such automatic recognition alternatively or additionally may include identification of regions having an expected geometrical relationship to the elongated reference structure, e.g. an expected symmetry and/or angular orientation. This utilizes the existing knowledge that further anatomical features typically have a specific anatomical arrangement with respect to the main orientation/direction of the elongated reference structure. For example, shoulders, ribs and hips are more or less symmetrical with respect to a spine, and are connected to the spine under a specific angular orientation. Such prior knowledge may help to detect these further anatomical features, e.g. in order to highlight them in the displayed volume slice.

In case of a recognized potential anatomical feature 53, 53', in order to make such proposed potential anatomical features 53, 53' immediately recognizable to the user, the processor arrangement 210 may further highlight the proposed potential anatomical features as recognized in the processed volume slice 51, 51' to be displayed by the display device 50 in 311, for example to assist the user in labeling the proposed potential anatomical features 53, 53' for landmark identification. Otherwise, the volume slice may be displayed in 309. Any suitable highlighting of the proposed potential anatomical features 53, 53' may be considered. For example, the processor arrangement 210 may in 311 generate a colour overlay over the proposed potential anatomical features 53, 53' as recognized in the processed volume slice 51, 51' such that the proposed potential anatomical feature stands out from the remainder of the displayed volume slice, thereby assisting the user to immediately recognize the proposed potential anatomical feature. In an embodiment, the processor arrangement 210 is further adapted to assign different colour overlays to different potential anatomical features 53, 53', for example based on a recognized shape of the potential anatomical feature, such that the processor arrangement 210 may suggest what type of anatomical feature potentially has been recognized, e.g. a shoulder blade, rib or hip bone, to further assist the user in identifying the potential anatomical feature. It is noted for the avoidance of doubt that such highlighting of the recognized potential anatomical feature 53, 53' by the processor arrangement 210 may be achieved in any suitable manner, e.g. by outlining the potential anatomical feature, by brightening the potential anatomical feature, and so on, such that it should be understood that the highlighting is not limited to colour overlays only.

In an embodiment, the processor arrangement 210 is further adapted to, upon displaying a volume slice 51, 51' with a recognized (highlighted) potential anatomical feature associated with the fetal reference structure 80, receive a user identification of the recognized anatomical feature in 313, i.e. the user confirms that the potential anatomical feature 53, 53' is an actual anatomical feature. For example, the user interface 120 may be configured to relay a user identification of a proposed potential anatomical feature to the processor arrangement 210 such that the processor arrangement 210 in 315 can label the anatomical feature 53, 53' in the displayed volume slice 51, 51' and add the anatomical label to the imaging volume stored in the data storage arrangement 220, e.g. by updating the stored imaging volume or by creating a new version of the imaging volume in a data storage device that may be the same or different to the data storage device storing the original imaging volume, which new version of the imaging volume includes the user-identified landmark labels.

In a straightforward embodiment, a user may identify a proposed anatomical feature using a keyboard of the user interface 120, in which the user identifies the proposed anatomical feature 53, 53' by means of a single keystroke, e.g. 's' for shoulders, 'r' for ribs, 'h' for hips and so on. The processor arrangement 220 may maintain a list of recognized anatomical features each associated with a single character, e.g. a single letter, and may check if a received keystroke from the user interface 120 corresponds to a character on this list. If this is the case, the processor arrangement 220 may update the imaging volume with a recognized landmark label as previously explained; if the keystroke is not recognized, the processor arrangement 220 may generate a warning signal or a warning message on the display device 50 to inform the user that the keystroke has not been recognized. It should be understood that this merely is a non-limiting example of how a user may provide an identification of a proposed anatomical feature to the ultrasound imaging apparatus 200. It will be immediately apparent to the skilled person that many other embodiments are equally feasible, such as for example a user typing in a label to be attached to the proposed anatomical feature 53, 53', a user selecting such a label from a drop-down menu displayed on the display device 50, and so on.

It may be checked in 317 if the user is to evaluate another volume slice along the fetal reference structure 80. If this is the case, the method 300 may revert back to 305 in which the processor arrangement 210 awaits the reception of a further user instruction of a translation of the reference point 81 along the fetal reference structure 80, such that a further volume slice corresponding to this new reference point 81 can be generated and displayed as previously explained. Otherwise, the method 300 may terminate in 319.

Using an embodiment of the ultrasound imaging system of the present invention, a user can more readily generate landmark labels for anatomical features within a captured imaging volume, which landmark labels may be used by the processor arrangement 210 to fit an articulated fetus model to the imaging volume using one or more received user identifications of a recognized anatomical feature, i.e. using one or more of the user-generated landmark labels. Such an articulated fetus model may be stored in the data storage arrangement 220; for example, an articulated fetus model may be stored together with the imaging volume in the data storage arrangement 220, e.g. in a compound image memory. It is noted that the fitting of an articulated fetus model to such an imaging volume is well-known per se and is therefore not explained in further detail for the sake of brevity only.

Fig. 6 schematically depicts an example embodiment of an ultrasound imaging system 1 in which the ultrasound imaging apparatus 200 is provided as a user console to which the ultrasound probe 10 comprising an ultrasound transducer array 11 is communicatively coupled, e.g. using an appropriate cable or the like. It should however be understood that at least parts of the ultrasound imaging system 1 may be distributed, e.g. provided as a remote service, in particular those elements for which the skilled person will understand that these elements are deployed for the processing of the sonographic data captured with the ultrasound transducer array 10.

In particular, Fig. 6 schematically depicts a block diagram of an example embodiment of the electronics that may be deployed to interface with and control the ultrasound transducer array 11 for the generation of ultrasound waves, e.g. ultrasound pulses, and reception of ultrasound echoes, e.g. pulse echoes, e.g. for diagnostic imaging purposes. At least part of these electronics may be embodied by the processor arrangement 210. Therefore, it should be understood that although these electronics are identified by different reference numerals, this does not necessarily mean that these electronics are distinct to the processor arrangement 210.

The ultrasound transducer array 11 may be coupled to a microbeam former 12, which may be located in the ultrasound probe 10 in some embodiments, which controls transmission and reception of signals by the ultrasound transducer cells 111 of the ultrasound transducer array 11. Microbeam formers are capable of at least partial beam forming of the signals received by groups or "patches" of transducer element tiles for instance as described in US patents US 5,997,479 (Savord et al.), US 6,013,032 (Savord), and US 6,623,432 (Powers et al.) The microbeam former 12 may be coupled by a probe cable, e.g. coaxial wire, to a terminal, e.g. an ultrasound imaging apparatus 200 such as a user console device or the like, which apparatus may comprise a transmit/receive (T/R) switch 16 which switches between transmission and reception modes and protects the main beam former 20 from high energy transmit signals when a microbeam former is not present or used and the transducer array is operated directly by the main system beam former 20.

The transmission of ultrasonic beams from the ultrasound transducer array 11 under control of the microbeam former 12 may be directed by a transducer controller 18 coupled to the microbeam former by the T/R switch 16 and the main system beam former 20, which receives input from the user's operation of the user interface 120 through control panel 38. One of the functions controlled by the transducer controller 18 is the direction in which beams are steered and focused. Beams may be steered straight ahead from (orthogonal to) the transducer array, or at different angles for a wider field of view. The transducer controller 18 may be coupled to control a voltage source 45 for the ultrasound transducer array 110. For instance, the voltage source 45 may set the DC and AC bias voltage(s) that are applied to CMUT (capacitive micro-machined ultrasound transducer) elements 111 of a CMUT array 11, e.g. to operate the CMUT elements in collapse mode, as is well-known per se although it should be understood that embodiments of the present invention are not limited to CMUT-based ultrasound probes 10 and that any suitable ultrasound probe may be used in the ultrasound imaging system 1 of the present invention. The transducer controller 18 may be further adapted to control the voltage supply 45 such as to switch the ultrasound transducer cells 130 to a low-power mode, e.g. in response to a temperature sensor signal indicative of the ultrasound transducer cells 130 reaching a critical temperature.

The partially beam-formed signals produced by the microbeam former 12 may be forwarded to the main beam former 20 where partially beam-formed signals from individual patches of transducer elements are combined into a fully beam-formed signal. For example, the main beam former 20 may have 128 channels, each of which receives a partially beam-formed signal from a patch of dozens or hundreds of ultrasound transducer cells 111 and/or from the individual ultrasound transducer elements of such ultrasound transducer cells 111. In this way the signals received by thousands of transducer elements of an ultrasound transducer array 100 can contribute efficiently to a single beam-formed signal.

The beam-formed signals are coupled to a signal processor 22, which may form part of the processor arrangement 210 as previously explained. The signal processor 22 can process the received echo signals in various ways, such as bandpass filtering, decimation, I and Q component separation, and harmonic signal separation which acts to separate linear and nonlinear signals so as to enable the identification of nonlinear (higher harmonics of the fundamental frequency) echo signals returned from tissue and microbubbles. The signal processor 22 optionally may perform additional signal enhancement such as speckle reduction, signal compounding, and noise elimination. The bandpass filter in the signal processor 22 may be a tracking filter, with its passband sliding from a higher frequency band to a lower frequency band as echo signals are received from increasing depths, thereby rejecting the noise at higher frequencies from greater depths where these frequencies are devoid of anatomical information.

The processed signals may be forwarded to a B-mode processor 26 and optionally to a Doppler processor 28, each of which may form part of the processor arrangement 210. The B-mode processor 26 employs detection of an amplitude of the received ultrasound signal for the imaging of structures in the body such as the tissue of organs and vessels in the body. B-mode images of structure of the body may be formed in either the harmonic image mode or the fundamental image mode or a combination of both for instance as described in US Patents US 6,283,919 (Roundhill et al.) and US 6,458,083 (Jago et al.)

The Doppler processor 28, if present, processes temporally distinct signals from tissue movement and blood flow for the detection of the motion of substances, such as the flow of blood cells in the image field. The Doppler processor typically includes a wall filter with parameters which may be set to pass and/or reject echoes returned from selected types of materials in the body. For instance, the wall filter can be set to have a passband characteristic which passes signal of relatively low amplitude from higher velocity materials while rejecting relatively strong signals from lower or zero velocity material.

This passband characteristic will pass signals from flowing blood while rejecting signals from nearby stationary or slowing moving objects such as the wall of the heart. An inverse characteristic would pass signals from moving tissue of the heart while rejecting blood flow signals for what is referred to as tissue Doppler imaging, detecting and depicting the motion of tissue. The Doppler processor may receive and process a sequence of temporally discrete echo signals from different points in an image field, the sequence of echoes from a particular point referred to as an ensemble. An ensemble of echoes received in rapid succession over a relatively short interval can be used to estimate the Doppler shift frequency of flowing blood, with the correspondence of the Doppler frequency to velocity indicating the blood flow velocity. An ensemble of echoes received over a longer period of time is used to estimate the velocity of slower flowing blood or slowly moving tissue.

The structural and motion signals produced by the B-mode (and Doppler) processor(s) are coupled to a scan converter 32 and a multiplanar reformatter 44. The scan converter 32 arranges the echo signals in the spatial relationship from which they were received in a desired image format. For instance, the scan converter may arrange the echo signal into a two dimensional (2D) sector-shaped format, or a pyramidal three dimensional (3D) image.

The scan converter can overlay a B-mode structural image with colors corresponding to motion at points in the image field with their Doppler-estimated velocities to produce a color Doppler image which depicts the motion of tissue and blood flow in the image field. The multiplanar reformatter 44 will convert echoes which are received from points in a common plane in a volumetric region of the body into an ultrasonic image of that plane, for instance as described in US Patent US 6,443,896 (Detmer). A volume renderer 42 converts the echo signals of a 3D data set into a projected 3D image as viewed from a given reference point as described in US Pat. 6,530,885 (Entrekin et al.)

The 2D or 3D images are coupled from the scan converter 32, multiplanar reformatter 44, and volume renderer 42 to an image processor 30 for further enhancement, buffering and temporary storage for display on an image display 50. The image processor 30 may form a part of the processor arrangement 210 and may further be adapted to control the visualization of volume slices as explained above. In addition to being used for imaging, the blood flow values produced by the Doppler processor 28 and tissue structure information produced by the B-mode processor 26 are coupled to a quantification processor 34. The quantification processor produces measures of different flow conditions such as the volume rate of blood flow as well as structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 50. The graphics processor 36 can also generate graphic overlays for display with the ultrasound images. These graphic overlays can contain standard identifying information such as patient name, date and time of the image, imaging parameters, and the like. For these purposes the graphics processor receives input from the control panel 38, such as patient name.

The user interface 120 may be coupled to the transmit controller 18 to control the generation of ultrasound signals from the transducer array 11 and hence the images produced by the transducer array 11 and the ultrasound system 1. The user interface 120 also may be coupled to the multiplanar reformatter 44 for selection and control of the planes of multiple multiplanar reformatted (MPR) images which may be used to perform quantified measures in the image field of the MPR images.

The above described embodiments of the method 300 may be realized by computer readable program instructions embodied on a computer readable storage medium having, when executed on a processor arrangement 200, cause the processor arrangement to implement the method 300. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In an embodiment, the ultrasound imaging apparatus 200 is adapted to retrieve the computer readable program instructions from such a computer readable storage medium and to create a new computer readable storage medium by storing the retrieved computer readable program instructions in the data storage arrangement 220, e.g. in a memory device or the like forming part of the data storage arrangement 220.

It should be noted that the above-mentioned embodiments illustrate rather than limit the invention, and that those skilled in the art will be able to design many alternative embodiments falling under the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An ultrasound imaging system (1) comprising:
a data storage arrangement (220) for storing ultrasound imaging data comprising an imaging volume of a fetus (8);
a processor arrangement (210) communicatively coupled to the data storage arrangement and responsive to a user interface (120); and
a display device (50) under control of said processor arrangement, wherein the processor arrangement is adapted to:
identify a fetal reference structure having an elongated shape (80) in said imaging volume;
define a reference point (81) on said fetal reference structure;
interpret a translation instruction from the user interface as a translation of said reference point along the elongation direction of the identified fetal reference structure;
display the imaging volume together with a volume slice (51, 51') of the imaging volume said volume slice being perpendicular to the elongation direction of the fetal reference structure on the display device, said volume slice coinciding with the translated reference point; **characterised in that** the processor arrangement is further adapted to
visualize said translation along said elongated shape of the fetal reference structure in the displayed imaging volume.

2. The ultrasound imaging system (1) of claim 1, wherein the fetal reference structure having an elongated shape (80) is the spine of the fetus (8).

3. The ultrasound imaging system (1) of claim 1 or 2, wherein the processor arrangement (210) is further adapted to:
recognize a potential anatomical feature (53, 53') of said fetus (8) in the volume slice (51, 51') coinciding with the translated reference point (81, 81'); and
highlight the recognized potential anatomical feature in the displayed volume slice.

4. The ultrasound imaging system (1) of claim 3, wherein the recognized potential anatomical feature (53, 53') is highlighted by a colour overlay over said recognized potential anatomical feature.

5. The ultrasound imaging system (1) of claim 3 or 4, wherein the processor arrangement (210) is further adapted to: receive a user identification of the recognized potential anatomical feature; and augmenting the imaging volume with the user identification.

6. The ultrasound imaging system (1) of claim 5, wherein the processor arrangement (210) is adapted to fit an articulated fetus model to said imaging volume using one or more received user identifications of a recognized potential anatomical feature (53, 53').

7. The ultrasound imaging system (1) of any of claims 1-6, further comprising an ultrasound probe (10) for obtaining the imaging volume of the fetus (8).

8. A method (300) of visualizing a volume slice (51, 51') of an imaging volume of a fetus (8) with an ultrasound imaging system (1), the method comprising:
retrieving (303) said imaging volume from a data storage arrangement (220);
identifying a fetal reference structure having an elongated shape (80) in said imaging volume;
defining a reference point (81) on said fetal reference structure;
interpreting (305) a translation instruction from the user interface as a translation of said reference point along the elongation direction of the identified fetal reference structure;
displaying (309, 311) the imaging volume together with a volume slice (51, 51') of the imaging volume said volume slice being perpendicular to the elongation direction of the fetal reference structure, said volume slice coinciding with the translated reference point (81'); **characterised in that** it further comprises:
visualizing said translation along said elongated shape of the fetal reference structure in the displayed imaging volume.

9. The method (300) of claim 8, further comprising:
recognizing (309) a potential anatomical feature (53, 53') of said fetus (8) in the volume slice (51, 51') coinciding with the translated reference point (81); and
highlighting (311) the recognized potential anatomical feature in the displayed volume slice.

10. The method (300) of claim 9, wherein the recognized potential anatomical feature (53, 53') is highlighted by a colour overlay over said recognized potential anatomical feature.

11. The method (300) of claim 9 or 10, further comprising:
receiving (313) a user identification of the recognized potential anatomical feature; and
augmenting (315) the imaging volume stored in the data storage arrangement (220) with the user identification.

12. The method (300) of claim 11, further comprising fitting an articulated fetus model to said imaging volume using one or more received user identifications of a recognized potential anatomical feature (53, 53 ').

13. A computer program product comprising a computer readable storage medium having computer readable program instructions embodied therewith for, when executed on a processor arrangement (210) of an ultrasound imaging system (1) of any of claims 1-7, cause the processor arrangement to implement the method (300) of any of claims 8-12.

## Patentansprüche

1. Ultraschallbildgebungssystem (1), umfassend: eine Datenspeicheranordnung (220) zum Speichern von Ultraschallbildgebungsdaten, die ein Bildgebungsvolumen eines Fötus (8) umfassen; eine Prozessoranordnung (210), die kommunikativ mit der Datenspeicher- Anordnung gekoppelt ist und auf eine Benutzerschnittstelle (120) reagiert; und eine Anzeigevorrichtung (50) unter der Steuerung der Prozessoranordnung, wobei die Prozessoranordnung angepasst ist zum: Identifizieren einer fötalen Referenzstruktur mit einer länglichen Form (80) im besagten Bildgebungsvolumen;
einen Referenzpunkt (81) auf der fötalen Referenzstruktur zu definieren; eine Übertragungsanweisung von der Benutzerschnittstelle als eine Übertragung des Referenzpunkts entlang der länglichen Erstreckungsrichtung der identifizierten fötalen Referenzstruktur zu interpretieren; Darstellen des Bildgebungsvolumens zusammen mit einem Volumenschnitt (51, 51') des Bildgebungsvolumens, wobei der Volumenschnitt senkrecht zur länglichen Erstreckungsrichtung der fötalen Referenzstruktur auf der Anzeigevorrichtung ist, wobei der besagte VolumenSchnitt mit dem übertragenen Referenzpunkt zusammenfällt; **dadurch gekennzeichnet, dass** die Prozessoranordnung weiter angepasst ist, um die Übertragung entlang der länglichen Form der fötalen Referenzstruktur in dem angezeigten Abbildungsvolumen optisch darzustellen.

2. Ultraschall-Bildgebungssystem (1) von Anspruch 1, wobei die fötale Referenz-Struktur, welche eine längliche Form (80) hat, das Rückgrat des Fötus (8) ist.

3. Ultraschallbildgebungssystem (1) nach Anspruch 1 oder 2, wobei die Prozessoranordnung (210) ferner angepasst ist zum: Erkennen eines potenziellen anatomischen Merkmals (53, 53') des Fötus (8) in der Volumenscheibe (51,51'), die mit dem verschobenen Referenzpunkt (81, 81') zusammenfällt; und das erkannte potenzielle anatomische Merkmal in der angezeigten Volumen-Scheibe markiert.

4. Ultraschallbildgebungssystem (1) nach Anspruch 3, wobei das erkannte potenzielle anatomische Merkmal (53, 53') durch eine Farbüberlagerung über dem erkannten potenziellen anatomischen Merkmal hervorgehoben wird.

5. Ultraschallbildgebungssystem (1) nach Anspruch 3 oder 4, wobei die Prozessoranordnung (210) ferner angepasst ist zum: Erhalten einer NutzerIdentifikation des erkannten potenziellen anatomischen Merkmals; und Steigern des Bildgebungsvolumens mit der Nutzeridentifikation.

6. Ultraschallbildgebungssystem (1) nach Anspruch 5, wobei die Prozessoranordnung (210) angepasst ist, um ein artikuliertes Fötusmodells unter Verwendung einer oder mehrerer empfangener Benutzeridentifikationen eines erkannten potenziellen anatomischen Merkmals (53, 53') an das Bildgebungsvolumen anzupassen.

7. Ultraschallbildgebungssystem (1) nach einem der Ansprüche 1 bis 6, ferner umfassend eine Ultraschallsonde (10) zum Erhalten des Bildgebungsvolumens des Fötus (8).

8. Verfahren (300) zum Visualisieren eines Volumenschnitts (51, 51') eines Bildgebungsvolumens eines Fötus (8) mit einem Ultraschallbildgebungssystem (1), wobei das Verfahren umfasst: Abrufen (303) des Bildgebungsvolumens aus einem Datenspeicher anordnen (220); Identifizieren einer fötalen Referenzstruktur mit einer länglichen Form (80) im besagten Bildgebungsvolumen;
Definieren eines Referenzpunkts (81) auf der fötalen Referenzstruktur; Interpretieren (305) einer Übertragungsanweisung von der Benutzerschnittstelle als eine Übertragung des Referenzpunkts entlang der länglichen Erstreckungsrichtung der identifizierten fötalen Referenzstruktur;
Darstellen (309, 311) des Bildgebungsvolumens zusammen mit einem Volumenschnitt (51, 51') des Bildgebungsvolumens, wobei der Volumenschnitt senkrecht zur länglichen Erstreckungsrichtung der fötalen Referenzstruktur ist, wobei der Volumenschnitt mit dem verschobenen Referenzpunkt (81'); **dadurch gekennzeichnet, dass** es ferner umfasst:
Sichtbarmachung der besagten Übertragung entlang der länglichen Form der fötalen Referenzstruktur in dem angezeigten Abbildungsvolumen.

9. Verfahren (300) nach Anspruch 8, welche weiterhin umfasst:
Erkennen (309) eines potenziellen anatomischen Merkmals (53, 53') des Fötus (8) in der Volumenscheibe (51, 51'), das mit dem verschobenen Referenzpunkt (81) zusammenfällt; und Hervorheben (311) des erkannten potenziellen anatomischen Merkmals in der angezeigten Volumenscheibe.

10. Verfahren (300) nach Anspruch 9, wobei das erkannte potenzielle anatomische Merkmal (53, 53') hervorgehoben wird durch eine Farbüberlagerung über dem erkannten potenziellen anatomischen Merkmal.

11. Verfahren (300) nach Anspruch 9 oder 10, ferner umfassend: Empfangen (313) einer Benutzeridentifikation des erkannten potenziellen anatomischen Merkmals; und
Steigern (315) des in der Datenspeicheranordnung (220) gespeicherten Bildgebungsvolumens mit der Benutzerkennung.

12. Verfahren (300) nach Anspruch 11, weiterhin umfassend die Anpassung eines artikulierten Fötusmodells unter Verwendung einer oder mehrerer empfangener Benutzeridentifikationen eines erkannten potenziellen anatomischen Merkmals (53, 53').

13. Computerprogrammprodukt, umfassend ein computerlesbares Speichermedium mit darauf verkörperten computerlesbaren Programmanweisungen, um bei Ausführung auf einer Prozessoranordnung (210) eines Ultraschallbildgebungssystems (1) nach einem der Ansprüche 1-7 die Prozessoranordnung zu veranlassen, das Verfahren (300) nach einem der Ansprüche 8-12 umzusetzen.

## Revendications

1. Système d'imagerie par ultrasons (1) comprenant:
un agencement de stockage de données (220) pour stocker des données d'imagerie par ultrasons comprenant un volume d'imagerie d'un fœtus (8);
un agencement de processeur (210) couplé de manière communicative à l'agencement de stockage de données et sensible à une interface utilisateur (120); et
un dispositif d'affichage (50) sous le contrôle dudit agencement de processeur, dans lequel l'agencement de processeur est adapté pour:
identifier une structure de référence fœtale ayant une forme allongée (80) dans ledit volume d'imagerie;
définir un point de référence (81) sur ladite structure de référence fœtale;
interpréter une instruction de translation provenant de l'interface utilisateur comme une translation dudit point de référence le long de la direction d'allongement de la structure de référence fœtale identifiée;
afficher le volume d'imagerie ainsi qu'une tranche de volume (51, 51') du volume d'imagerie, ladite tranche de volume étant perpendiculaire à la direction d'allongement de la structure de référence fœtale sur le dispositif d'affichage, ladite tranche de volume coïncidant avec le point de référence translaté;
**caractérisé en ce que** l'agencement de processeur est en outre adapté pour Visualiser ladite translation le long de ladite forme allongée de la structure de référence fœtale dans le volume d'imagerie affiché.

2. Système d'imagerie par ultrasons (1) selon la revendication 1, dans lequel la structure de référence fœtale ayant une forme allongée (80) est la colonne vertébrale du fœtus (8).

3. Système d'imagerie par ultrasons (1) selon la revendication 1 ou 2, dans lequel l'agencement de processeur (210) est en outre adapté pour:
reconnaître une caractéristique anatomique potentielle (53, 53') dudit fœtus (8) dans la tranche de volume (51, 51') coïncidant avec le point de référence translaté (81, 81'); et
mettre en évidence la caractéristique anatomique potentielle reconnue dans la tranche de volume affichée.

4. Système d'imagerie par ultrasons (1) selon la revendication 3, dans lequel la caractéristique anatomique potentielle reconnue (53, 53') est mise en évidence par une superposition de couleurs sur ladite caractéristique anatomique potentielle reconnue.

5. Système d'imagerie par ultrasons (1) selon la revendication 3 ou 4, dans lequel l'agencement de processeur (210) est en outre adapté pour:
recevoir une identification utilisateur de la caractéristique anatomique potentielle reconnue; et
augmenter le volume d'imagerie avec l'identification de l'utilisateur.

6. Système d'imagerie par ultrasons (1) selon la revendication 5, dans lequel l'agencement de processeur (210) est adapté pour ajuster un modèle de fœtus articulé audit volume d'imagerie en utilisant une ou plusieurs identifications d'utilisateur reçues d'une caractéristique anatomique potentielle reconnue (53, 53').

7. Système d'imagerie par ultrasons (1) selon l'une quelconque des revendications 1 à 6, comprenant en outre une sonde à ultrasons (10) pour obtenir le volume d'imagerie du fœtus (8).

8. Procédé (300) de visualisation d'une tranche de volume (51, 51') d'un volume d'imagerie d'un fœtus (8) avec un système d'imagerie par ultrasons (1), le procédé comprenant:
la récupération (303) dudit volume d'imagerie à partir d'un dispositif de stockage de données (220);
identifier une structure de référence fœtale ayant une forme allongée (80) dans ledit volume d'imagerie;
définir un point de référence (81) sur ladite structure de référence fœtale;
interpréter (305) une instruction de translation provenant de l'interface utilisateur comme une translation dudit point de référence le long de la direction d'allongement de la structure de référence fœtale identifiée;
afficher (309, 311) le volume d'imagerie ainsi qu'une tranche de volume (51, 51') du volume d'imagerie, ladite tranche de volume étant perpendiculaire à la direction d'allongement de la structure de référence fœtale, ladite tranche de volume coïncidant avec le point de référence translaté (81'); **caractérisé en ce qu'**il comprend en outre:
la visualisation de ladite translation le long de ladite forme allongée de la structure de référence fœtale dans le volume d'imagerie affiché.

9. Procédé (300) selon la revendication 8, comprenant en outre:
reconnaître (309) une caractéristique anatomique potentielle (53, 53') dudit fœtus (8) dans la tranche de volume (51, 51') coïncidant avec le point de référence translaté (81); et
mettre en évidence (311) la caractéristique anatomique potentielle reconnue dans la tranche de volume affichée.

10. Procédé (300) selon la revendication 9, dans lequel la caractéristique anatomique potentielle reconnue (53, 53') est mise en évidence par une superposition de couleurs sur ladite caractéristique anatomique potentielle reconnue.

11. Procédé (300) selon la revendication 9 ou 10, comprenant en outre:
recevoir (313) une identification d'utilisateur de la caractéristique anatomique potentielle reconnue; et
augmenter (315) le volume d'imagerie stocké dans l'agencement de stockage de données (220) avec l'identification de l'utilisateur.

12. Procédé (300) selon la revendication 11, comprenant en outre l'adaptation d'un modèle de fœtus articulé audit volume d'imagerie en utilisant une ou plusieurs identifications d'utilisateur reçues d'une caractéristique anatomique potentielle reconnue (53, 53').

13. Produit de programme informatique comprenant un support de stockage lisible par ordinateur ayant des instructions de programme lisibles par ordinateur incorporées avec celui-ci pour, lorsqu'il est exécuté sur un agencement de processeur (210) d'un système d'imagerie par ultrasons (1) de l'une quelconque des revendications 1 à 7, amener l'agencement de processeur à mettre en œuvre le procédé (300) de l'une quelconque des revendications 8 à 12.
